# EUROPEAN PATENT APPLICATION

(11) **EP 4 465 660 A1**
(43) Date of publication of application: **20.11.2024**
(21) Application number: 24176149.3
(22) Date of filing: 16.05.2024
(51) Int. Cl.: H04R 25/00

(54) **HEARING ASSISTANCE DEVICES WITH DYNAMIC GAIN CONTROL BASED ON DETECTED CHEWING OR SWALLOWING**

(30) Priority: 17.05.2023 US 202363467145 P
(71) Applicant: Starkey Laboratories, Inc., Eden Prairie, MN 55344 (US)
(72) Inventor: Bhowmik, Achintya Kumar, Cupertino (US); Austin, William F., Eden Prairie (US)
(74) Representative: Vossius & Partner Patentanwälte Rechtsanwälte mbB

(57) **Abstract**

Embodiments herein relate to hearing assistance systems and devices. In an embodiment, a hearing assistance device is included having a control circuit, a microphone, a motion sensor, a receiver, and a sound processor. The hearing assistance device is configured to output sound to a wearer of the hearing assistance device with the receiver based on sound detected with the microphone, detect chewing of the wearer by evaluating signals from the microphone and/or the motion sensor, and modulate a gain value of the sound processor for sounds output through the receiver based on the chewing detected. Other embodiments are also included herein.

## Description

### Field

Embodiments herein relate to hearing assistance systems and devices.

### Background

Hearing assistance devices can provide tremendous benefit to those with some degree of hearing loss or perceived hearing difficulty. Modern hearing assistance devices are extremely powerful and have many capabilities.

Because of the substantial benefits, individuals typically wear hearing assistance devices continuously throughout their day encompassing many different activities including, working, socializing, exercising, eating, and the like. Such different activities may include different sound environments, different activity levels, and different demands on the hearing assistance device. However, it is desirable for hearing assistance devices to perform optimally regardless of the activity taking place.

### Summary

Embodiments herein relate to hearing assistance systems and devices. In a first aspect, a hearing assistance device can be included having a control circuit, a microphone, a motion sensor, a receiver, and a sound processor. The hearing assistance device can be configured to output sound to a wearer of the hearing assistance device with the receiver based on sound detected with the microphone, detect chewing of the wearer by evaluating signals from the microphone and/or the motion sensor, and modulate a gain value of the sound processor for sounds output through the receiver based on the chewing detected.

In a second aspect, in addition to one or more of the preceding or following aspects, or in the alternative to some aspects, the hearing assistance device can be configured to reduce the gain value of the sound processor for sounds detected with the microphone and then output through the receiver based on the chewing detected.

In a third aspect, in addition to one or more of the preceding or following aspects, or in the alternative to some aspects, the hearing assistance device can be configured to receive an input regarding a degree of gain value reduction when the chewing can be detected.

In a fourth aspect, in addition to one or more of the preceding or following aspects, or in the alternative to some aspects, the hearing assistance device can be configured to adjust the gain value of the sound processor greater than 20 times per second.

In a fifth aspect, in addition to one or more of the preceding or following aspects, or in the alternative to some aspects, the hearing assistance device can be configured to detect chewing of the wearer by detecting a predetermined pattern within signals from the microphone and/or the motion sensor.

In a sixth aspect, in addition to one or more of the preceding or following aspects, or in the alternative to some aspects, the hearing assistance device can be configured to execute a binary classification for the chewing detection on a per time unit basis.

In a seventh aspect, in addition to one or more of the preceding or following aspects, or in the alternative to some aspects, the hearing assistance device can be configured to adjust a weighting factor for detection of the chewing based on a detected posture.

In an eighth aspect, in addition to one or more of the preceding or following aspects, or in the alternative to some aspects, the hearing assistance device can be configured to adjust a weighting factor for detection of the chewing based on detection that the device wearer is seated.

In a ninth aspect, in addition to one or more of the preceding or following aspects, or in the alternative to some aspects, the hearing assistance device can be configured to characterize an ambient sound environment and set a degree of gain value modulation based on the same.

In a tenth aspect, in addition to one or more of the preceding or following aspects, or in the alternative to some aspects, the sound processor can include an amplifier.

In an eleventh aspect, in addition to one or more of the preceding or following aspects, or in the alternative to some aspects, the sound processor can include a digital signal processor.

In a twelfth aspect, in addition to one or more of the preceding or following aspects, or in the alternative to some aspects, the motion sensor can include an accelerometer.

In a thirteenth aspect, in addition to one or more of the preceding or following aspects, or in the alternative to some aspects, the receiver can include a speaker.

In a fourteenth aspect, in addition to one or more of the preceding or following aspects, or in the alternative to some aspects, the hearing assistance device can include at least one selected from the group consisting of a RIC hearing aid and a custom hearing aid.

In a fifteenth aspect, a hearing-assistance system can be included having a first hearing assistance device and a second hearing assistance device. The first hearing assistance device can include a first control circuit, a first microphone, a first motion sensor, and a first speaker. The second hearing assistance device can include a second control circuit, a second microphone, a second motion sensor, and a second speaker. The hearing-assistance system can be configured to output sound to a wearer of the hearing-assistance system based on sound detected with the first microphone and/or the second microphone, detect chewing of a device wearer by evaluating signals from the microphones and/or the motion sensors, and modulate a gain value for sounds detected with the microphones and then output through the speakers based on the detected chewing.

In a sixteenth aspect, in addition to one or more of the preceding or following aspects, or in the alternative to some aspects, the hearing-assistance system can be configured to reduce the gain value for sounds detected with the first microphone and/or the second microphone and then output through the first speaker and/or the second speaker based on the chewing detected.

In a seventeenth aspect, in addition to one or more of the preceding or following aspects, or in the alternative to some aspects, the hearing-assistance system can be configured to receive an input regarding a degree of gain value reduction when the chewing can be detected.

In an eighteenth aspect, in addition to one or more of the preceding or following aspects, or in the alternative to some aspects, the hearing-assistance system can be configured to adjust the gain value greater than 20 times per second.

In a nineteenth aspect, in addition to one or more of the preceding or following aspects, or in the alternative to some aspects, the hearing-assistance system can be configured to detect chewing of the wearer by detecting a predetermined pattern within signals from the microphones and/or the motion sensors.

In a twentieth aspect, in addition to one or more of the preceding or following aspects, or in the alternative to some aspects, the hearing-assistance system can be configured to execute a binary classification for the chewing detection on a per time unit basis.

In a twenty-first aspect, in addition to one or more of the preceding or following aspects, or in the alternative to some aspects, the hearing-assistance system can be configured to adjust a weighting factor for detection of the chewing based on a detected posture.

In a twenty-second aspect, in addition to one or more of the preceding or following aspects, or in the alternative to some aspects, the hearing-assistance system can be configured to adjust a weighting factor for detection of the chewing based on detection that the device wearer can be seated.

In a twenty-third aspect, in addition to one or more of the preceding or following aspects, or in the alternative to some aspects, the hearing-assistance system can be configured to characterize an ambient sound environment and set a degree of gain value modulation based on the same.

In a twenty-fourth aspect, in addition to one or more of the preceding or following aspects, or in the alternative to some aspects, wherein gain values for the first hearing assistance device and the second hearing assistance device can be adjusted independently.

In a twenty-fifth aspect, in addition to one or more of the preceding or following aspects, or in the alternative to some aspects, the hearing-assistance system can include at least one selected from the group consisting of a RIC hearing aid and a custom hearing aid.

In a twenty-sixth aspect, a method of dynamically adjusting gain values for a hearing assistance device can be included. The method including outputting sound to a wearer of a wearer of a hearing assistance device based on sound detected with a microphone, detecting chewing of the device wearer by evaluating signals from the microphone and/or the motion sensor, and modulating a gain value of a sound processor for sounds output through the receiver based on the chewing detected.

In a twenty-seventh aspect, in addition to one or more of the preceding or following aspects, or in the alternative to some aspects, the method can further include reducing the gain value of the sound processor for sounds detected with the microphone and then output through the receiver based on the chewing detected.

In a twenty-eighth aspect, in addition to one or more of the preceding or following aspects, or in the alternative to some aspects, the method can further include receiving an input regarding a degree of gain value reduction when the chewing can be detected.

In a twenty-ninth aspect, in addition to one or more of the preceding or following aspects, or in the alternative to some aspects, the method can further include adjusting the gain value of the sound processor greater than 20 times per second.

In a thirtieth aspect, in addition to one or more of the preceding or following aspects, or in the alternative to some aspects, the method can further include detecting chewing of the wearer by detecting a predetermined pattern within signals from the microphone and/or the motion sensor.

In a thirty-first aspect, in addition to one or more of the preceding or following aspects, or in the alternative to some aspects, the method can further include executing a binary classification for the chewing detection on a per time unit basis.

In a thirty-second aspect, in addition to one or more of the preceding or following aspects, or in the alternative to some aspects, the method can further include adjusting a weighting factor for detection of the chewing based on a detected posture.

In a thirty-third aspect, in addition to one or more of the preceding or following aspects, or in the alternative to some aspects, the method can further include adjusting a weighting factor for detection of the chewing based on detecting that the device wearer can be seated.

In a thirty-fourth aspect, in addition to one or more of the preceding or following aspects, or in the alternative to some aspects, the method can further include characterizing an ambient sound environment and setting a degree of gain value modulation based on the same.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims and their legal equivalents.

### Brief Description of the Figures

Aspects may be more completely understood in connection with the following figures (FIGS.), in which:
FIG. 1 is a schematic view of an individual wearing a hearing assistance device herein and eating in accordance with various embodiments herein.
FIG. 2 is a schematic view of a hearing assistance device in accordance with various embodiments herein.
FIG. 3 is a schematic view of a hearing assistance device within the ear in accordance with various embodiments herein.
FIG. 4 is a schematic view of signal generation associated with chewing in accordance with various embodiments herein.
FIG. 5 is a schematic view of signal generation associated with chewing in accordance with various embodiments herein.
FIG. 6 is a diagram of chewing detection operations in accordance with various embodiments herein.
FIG. 7 is a diagram of gain value modulation in accordance with various embodiments herein.
FIG. 8 is a schematic view of an accessory device in accordance with various embodiments herein.
FIG. 9 is a schematic view of a device wearer assuming a seated position in accordance with various embodiments herein.
FIG. 10 is a schematic view of hearing assistance system data network in accordance with various embodiments herein.
FIG. 11 is a block diagram of components of a hearing assistance device in accordance with various embodiments herein.
FIG. 12 is a schematic view of a hearing assistance device in accordance with various embodiments herein.
FIG. 13 is a schematic view of a hearing assistance device within the ear in accordance with various embodiments herein.

While embodiments are susceptible to various modifications and alternative forms, specifics thereof have been shown by way of example and drawings, and will be described in detail. It should be understood, however, that the scope herein is not limited to the particular aspects described. On the contrary, the intention is to cover modifications, equivalents, and alternatives falling within the spirit and scope herein.

### Detailed Description

As referenced above, individuals typically wear hearing assistance devices continuously throughout their day encompassing many different activities including, working, socializing, exercising, eating, and the like. It is desirable for hearing assistance devices to perform optimally regardless of the activity taking place.

The gain of a hearing assistance device can be thought of as the difference between the level of a sound entering a microphone of the hearing assistance device and the amplified level of the sound exiting a speaker (which may be part of a receiver) of the hearing assistance device. Appropriate gain values ensure that the hearing aid user can hear sounds comfortably and clearly across different frequencies and input levels while also preventing acoustic feedback, which is a squeal or screech that occurs when the amplified sound from the speaker leaks back to the microphone and creates a positive feedback loop.

Some types of hearing assistance devices form a seal with the inner surface of the ear canal that acoustically separates the microphone(s) and the speaker(s). When this seal is disrupted, more sound can leak back from the speaker to the microphone potentially creating feedback issues. Chewing and/or related jaw motion such as that associated with eating, drinking, mastication, etc. can cause movement of the inner surface of the ear canal which can disrupt the seal between the hearing assistance device and the inner surface of the ear canal potentially leading to feedback issues.

However, systems and devices herein can reduce feedback issues associated with chewing and/or related jaw motions. In specific, systems and devices herein can detect chewing and/or related jaw motion using sensors as described herein and then dynamically adjust the gain parameters of the hearing assistance device in order to reduce or eliminate any feedback issues that may otherwise occur. Such adjustments can be executed by the system or device automatically such that the device wearer is unaware of the dynamical changes made to the gain parameters.

As an example, a hearing assistance device is included in various embodiments herein having a control circuit, a microphone, a motion sensor, a receiver, and a sound processor. The hearing assistance device can be configured to output sound to a wearer of the hearing assistance device via the receiver based on sound detected with the microphone. Further, the hearing assistance device can detect chewing of the wearer by evaluating signals from the microphone and/or the motion sensor and then modulate a gain value of the sound processor for sounds output through the receiver based on the chewing detected.

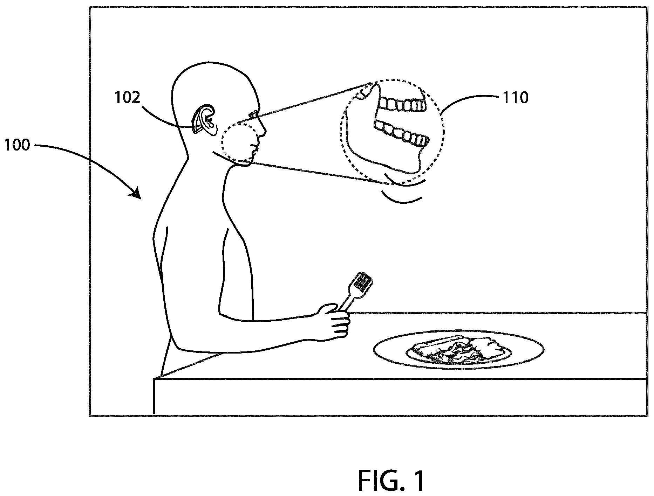

Referring now to FIG. 1, a schematic view is shown of a device wearer 100 with a hearing assistance device 102. In FIG. 1, the device wearer 100 is shown eating. FIG. 1 also shows an enlarged view of the device wearer's mouth 110. As will be described further below, the hearing assistance device 102 can be configured to output sound to the device wearer 100 with a receiver (which can include a speaker) based on sound detected with a microphone. However, the hearing assistance device 102 can be configured to detect chewing (or similar jaw motion) of the wearer by evaluating signals from a microphone and/or a motion sensor. Exemplary techniques for detecting chewing will be described further below. Then, in various embodiments, the hearing assistance device 102 can be configured to modulate a gain value of a sound processor for sounds output through the receiver based on the chewing detected. For example, the gain value can be reduced when chewing is detected to reduce the chance of feedback issues occurring.

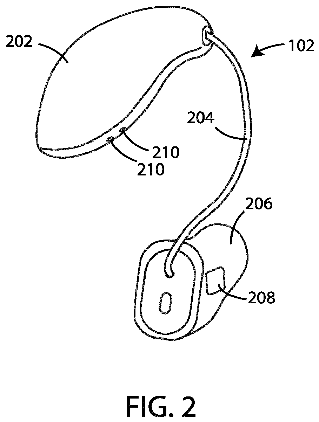

Referring now to FIG. 2, a schematic view of a hearing assistance device 102 is shown in accordance with various embodiments herein. In this example, the hearing assistance device 102 includes a housing 202. The hearing assistance device 102 also includes a cable 204. The hearing assistance device 102 also includes a receiver 206. The hearing assistance device 102 also includes a sensor 208. The hearing assistance device 102 also includes electrical contacts 210.

It will be appreciated that while FIG. 2 illustrates one type of hearing assistance device (or ear-wearable device) consistent with embodiments herein, many other types of hearing assistance devices are also contemplated. The term "hearing assistance device" as used herein can include devices that can aid a person with impaired hearing. The term "hearing assistance device" shall also refer to devices that can produce optimized or processed sound for persons with normal hearing. Hearing assistance devices herein can include, but are not limited to, behind-the-ear (BTE), in-the ear (ITE), in-the-canal (ITC), invisible-in-canal (IIC), receiver-in-canal (RIC), receiver in-the-ear (RITE) and completely-in-the-canal (CIC) type hearing assistance devices. In various embodiments, hearing assistance devices wherein can include those wherein a portion of the device forms a seal with an inner surface of the ear, such as the ear canal. In some embodiments, the hearing assistance device can be a hearing aid falling under 21 C.F.R. 9 801.420. In some embodiments, the hearing assistance device can be an over-the-counter (OTC) hearing aid. In another example, the hearing assistance device can include one or more Personal Sound Amplification Products (PSAPs). In another example, the hearing assistance device can include one or more "hearable" devices that provide various types of functionality. In other examples, hearing assistance devices can include other types of devices that are wearable in, on, or in the vicinity of the user's ears.

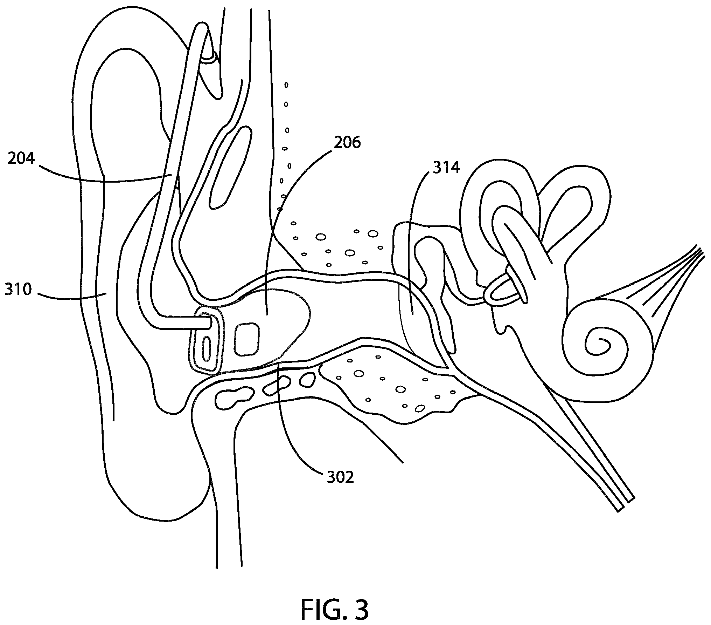

Referring now to FIG. 3, a schematic view of a hearing assistance device 102 within the ear is shown in accordance with various embodiments herein. The ear includes the ear canal 302, the pinna 310, and the tympanic membrane 314. The hearing assistance device 102 includes a cable 204 and a receiver 206. The receiver 206 is disposed within the ear canal 302 and portions of the hearing assistance device 102 form a seal with the inner surfaces of the ear canal 302.

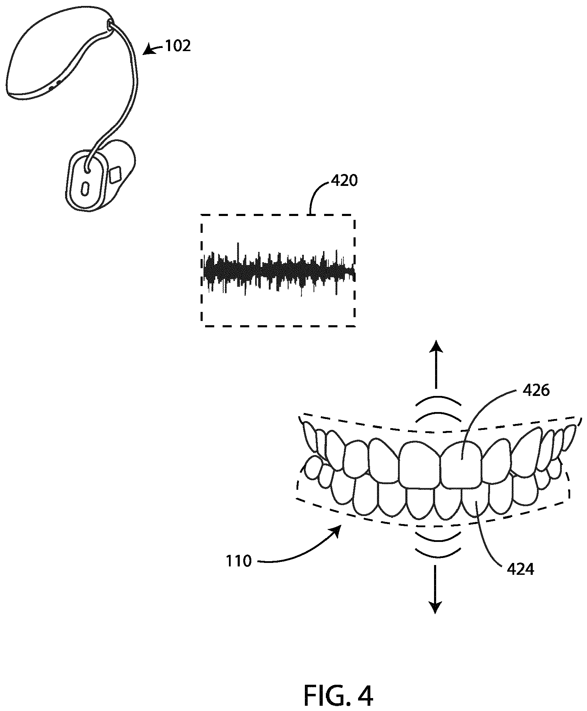

Referring now to FIG. 4, a schematic view of signal generation associated with chewing is shown in accordance with various embodiments herein. The mouth 110 of the device wearer includes lower teeth 424 and upper teeth 426 which move relative to one another during chewing based on movement of the jaw. FIG. 4 also shows a hearing assistance device 102. The hearing assistance device 102 can include various sensors generating sensor signals 420. The sensor signals 420 can be processed to determine that the device wearer is chewing. As used herein, the term "chewing" will also include other activities including similar jaw motion unless the context dictates otherwise.

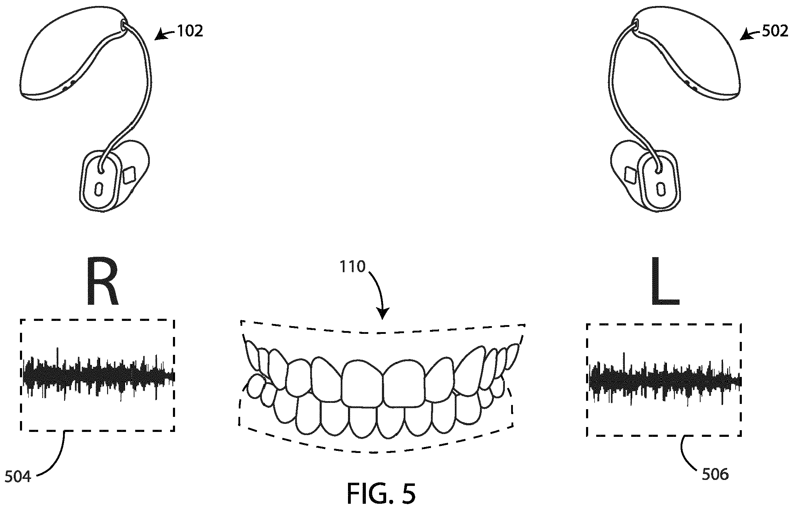

In some embodiments, a device wearer may only be wearing a single hearing assistance device. However, it will be appreciated that in many embodiments a device wearer will be wearing a pair of hearing assistance devices with one in each ear. Referring now to FIG. 5, a schematic view of signal generation associated with chewing is shown in accordance with various embodiments herein. As before, the mouth 110 of the device wearer is shown. FIG. 5 shows a first hearing assistance device 102 generating right side signals 504 and a second hearing assistance device 502 generating left side signals 506.

In some embodiments, the first hearing assistance device 102 and the second hearing assistance device 502 can operate independently from one another with respect to chewing detection and/or gain control herein. However, in other embodiments, the first hearing assistance device 102 and the second hearing assistance device 502 can operate in a coordinated matter with each other with respect to chewing detection and/or gain control herein. By way of example, in some embodiments the first hearing assistance device 102 and the second hearing assistance device 502 are in signal communication with one another and/or with an accessory device. In some embodiments, the first hearing assistance device 102 and the second hearing assistance device 502 are configured to send and/or receive sensor signals (such as microphone signals and/or motion sensor signals). In some embodiments, the first hearing assistance device 102 and the second hearing assistance device 502 are configured to send and/or receive indications of chewing detection. In some embodiments, the first hearing assistance device 102 and the second hearing assistance device 502 are configured to send and/or receive commands regarding gain modulation, such as reducing gain when chewing is detected and/or returning gain to an initial or default level within chewing is not detected. In some embodiments, a consensus approach can be used such that a hearing assistance device will only modulate gain for chewing detection when both the devices detect chewing.

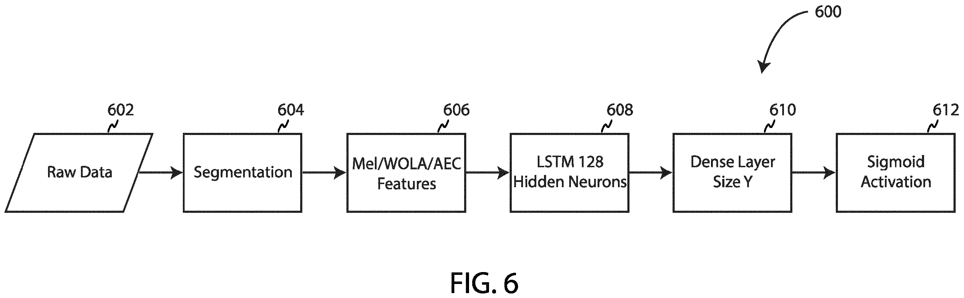

It will be appreciated that systems and/or devices herein can execute various data processing and/or signal processing operations to detect chewing. Such operations can be performed on hearing assistance devices, on accessory devices, on computing resources in the cloud, and/or can be distributed across multiple of such sites. Referring now to FIG. 6, a diagram of data processing operations 600 is shown in accordance with various embodiments herein. Raw sensor data 602 can be subjected to an operation of segmentation 604. Segmentation can include breaking up the signal into discrete units cover a specific time span. Various time spans can be used, however in some embodiments the time span can be on the order of millisecond, such as 5, 10, 25, 50, 100, 150, 200, 300 milliseconds, or an amount a time falling within a range between any of the foregoing.

The segmented signal data can then be used in operations of feature extraction and signal processing 606. Feature extraction and signal processing 606 herein can include the use of Mel frequency cepstral coefficient (MFCC) feature extraction. Feature extraction and signal processing 606 can also include WOLA (weighted-overlap add) algorithm signal processing creating WOLA bands. Feature extraction and signal processing 606 can also include an acoustic echo cancelation (AEC) operation.

The processed data (such as WOLA bands) can then be evaluated 608 in an artificial intelligence (AI) system comprising a recurrent neural network, such as a long short-term memory (LSTM) architecture using a plurality of neuron units. The data fed into the LSTM can by an M*N AEC matrix, where M is a fixed number of time points and N is a number of WOLA/Mel features. In some embodiments, two layers of LSTM are used with 128 hidden layers in the first layer and 256 layers in the second. A supervised learning approach can be used with labeled learning data including recordings of chewing or the like. However, semi-supervised learning approaches can also be used. The LSTM recurrent neural network output can then be fed into a dense layer 610 (a fully connected layer that follows LSTM layers and is used for outputting a prediction). The output of the dense layer can then be subject to a sigmoid activation 612 function as the last layer. The final output can be a probability per time period. For example, the final output can be probability per time period of chewing taking place. In some embodiments, the probability can be compared with a threshold value and if the threshold value is exceeded then the time period or frame can be taken as indicative of chewing taking place. It will be appreciated that the described approach for data processing / signal processing is only one specific example and many variations on this approach are contemplated herein.

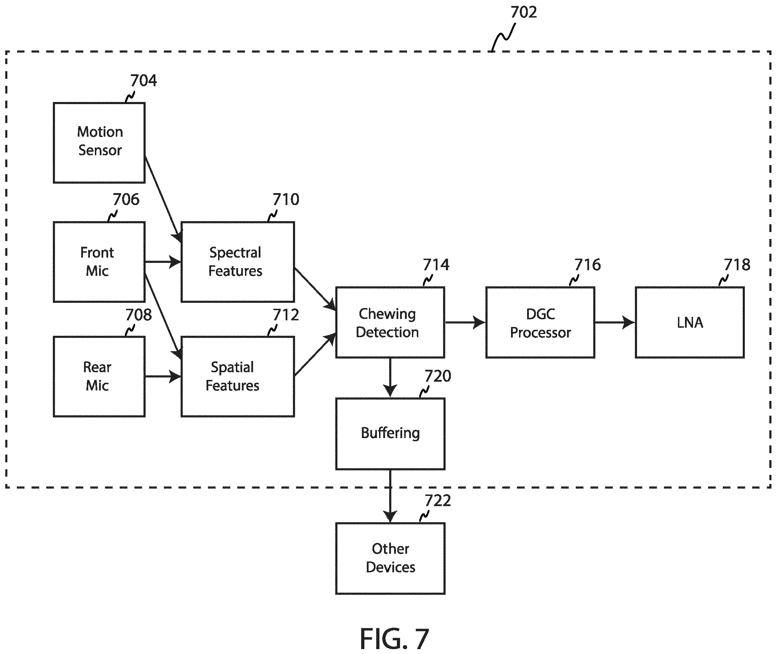

Referring now to FIG. 7, a diagram of an exemplary process for detecting chewing using data from a microphone and/or a motion sensor is shown in accordance with various embodiments herein. In this example, various operations are performed at the level of an ear-worn device, an external device, and/or the cloud.

A front microphone 706 is used to generate signals representative of sound along with a rear microphone 708. In this example, the signals from the front microphone 706 are then processed to evaluate/extract spectral and/or temporal features 710 therefrom. In some embodiments, signals from a motion sensor 704 can also be processed to evaluate/extract spectral and/or temporal features 710 therefrom. Many different spectral and/or temporal features can be evaluated/extracted including, but not limited to, those shown in the following table.

**TABLE 1**

| **Feature Name** |
|---|
| Zero-Crossing Rate |
| Periodicity Strength |
| Short Time Energy |
| Spectral Centroid |
| Spectral Centroid Mean |
| Spectral Bandwidth |
| Spectral Roll-off |
| Spectral Flux |
| High-/Low-Frequency Energy Ratio |
| High-Frequency Slope |
| Low-Frequency Slope |
| Absolute Magnitude Difference Function |
| Spectral Flux at High Frequency |
| Spectral Flux at Low Frequency |
| Periodicity Strength |
| Low Frequency Envelope Peakiness |
| Onset Rate |

Spectral and/or temporal features that can be utilized from signals of a single-mic can include, but are not limited to, HLF (the relative power in the high-frequency portion of the spectrum relative to the low-frequency portion), SC (spectral centroid), LS (the slope of the power spectrum below the Spectral Centroid), PS (periodic strength), and Envelope Peakiness (a measure of signal envelope modulation).

In embodiments with at least two microphones, one or more of the following signal features can be used to detect chewing using the spatial information between two microphones.
- MSC: Magnitude Squared Coherence.
- ILD: level difference
- IPD: phase difference

The MSC feature can be used to determine whether a source is a point source or distributed. The ILD and IPD features can be used to determine the direction of arrival of the sound. Chewing sounds are located at a particular location relative to the microphones on the device. Also chewing sounds are distributed and caused by chewing activities in the whole mouth (in contrast, for example, speech is mostly emitted from the lips.)

It will be appreciated that when at least two microphones are used that have some physical separation from one another that the signals can then be processed to derive/extract/utilize spatial information 712. For example, signals from the front microphone 706 and the rear microphone 708 can be correlated in order to extract those signals representing sound with a point of origin falling in an area associated with the inside of the device wearer. As such, this operation can be used to separate signals associated with external noise and external speech from signals associated with chewing sounds of the device wearer.

Using data associated with the sensor signals directly, spectral features of the sensor signals, and/or data associated with spatial features, an operation can be executed in order to detect 714 chewing. In some embodiments, a technique can be used to detect chewing as described in FIG. 6. However, it will be appreciated that various machine learning techniques can be applied in order to take the signals from sensors as described herein and determine whether or not chewing is happening. In specific, machine learning techniques related to classification can be applied in order to determine whether or not chewing is occurring. In various embodiments, the hearing assistance device can be configured to detect chewing of the wearer by detecting a predetermined pattern within signals from the microphone and/or the motion sensor. Machine learning techniques that can be applied can include supervised learning, unsupervised learning, and reinforcement learning. In some embodiments, techniques applied can include one or more of artificial neural networks, convolutional neural networks, K nearest neighbor techniques, decision trees, support vector machines, or the like. In some embodiments herein, a multi-node decision tree can be used to reach a binary result (e.g. binary classification) on whether the individual is chewing or not.

In some embodiments, signals or other data derived therefrom can be divided up into discrete time units (such as periods of milliseconds, seconds, minutes, or longer) and the system can perform binary classification (e.g., "chewing" or "not chewing") regarding whether the individual was chewing during that discrete time unit. As an example, in some embodiments, signal processing or evaluation operations herein to identify chewing events can include binary classification for chewing detection on a per second basis. Aspects of chewing detecting are described in PCT Publ. Appl. No. WO2022/026557, the content of which is herein incorporated by reference in its entirety.

After chewing detection 714, information regarding the same can be fed into a DGC (dynamic gain control) processor 716 (which could be an independent processor or could be integrated into another processor herein). Output from the DGC processor 716 can then be used to control and/or adjust a gain value of an amplifier, such as a low-noise amplifier (LNA) 718 to implement gain modulation herein. It will be appreciated that the gain value can be adjusted with respect to an initial or default level of gain. In some embodiments, the gain value can be adjusted (up or down) by 0.1 dB, 1 dB, 2 dB, 3 dB, 4 dB, 5 dB, 10 dB, 15 dB, 20 dB, 30 dB or more, or by an amount falling within a range between any of the foregoing. In some embodiments, the gain value can be adjusted within one or more specific frequency ranges. In some embodiments, the gain value can be adjusted at frequencies including those typical for feedback. Gain modulation can be particularly helpful in high-gain scenarios. In various embodiments, gain modulation herein can be performed when the default level of gain at one or more frequencies is 50, 60, 70, 80, 90, or 100 dB or more. As merely one example, gain modulation herein could include a drop from 65 dB down to 45 dB. However, many different magnitudes of gain modulation are contemplated herein.

In some embodiments, data regarding chewing events can be buffered 720 at the level of the ear-worn device 702 before being passed on to another device 722. While FIG. 7 illustrates detection of chewing at the level of the ear-worn device 702, it will be appreciated that in some embodiments signal data can be passed from the ear-worn device 702 onto another device and detection can occur there.

While FIG. 7 exemplifies a scenario with an ear-worn device having two microphones (a front microphone and a rear microphone), it will be appreciated that similar techniques can be applied to ear-worn devices having different numbers of microphones. In addition, in some embodiments, similar techniques can be applied to a system including two ear-worn devices (e.g., a right and a left device) with the correlation (spatial isolation) occurring between signals of each ear-worn device (versus between signals of microphones of the same ear-worn device).

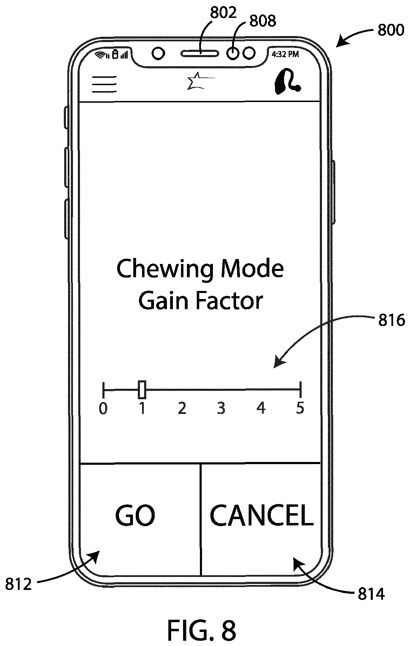

Accessory devices can be used in accordance with devices and systems herein and can include various components. Referring now to FIG. 8, a schematic view of an accessory device 800 is shown in accordance with various embodiments herein. The accessory device 800 includes a speaker 802. The accessory device 800 also includes a camera 808. The accessory device 800 also includes a first user input object 812. The accessory device 800 also includes a second user input object 814.

In this example, the ear-wearable device can be configured to utilize the accessory device 800 to receive input from the device wearer regarding a desired level of gain modulation for preventing chewing related feedback issues. For example, a user input element 816 (in this case in the form of a slider) can be used to collect information from the device wearer (or another individual) regarding a desired level of gain modulation. In this example, a level of "0" would indicate no gain modulation whereas a level of "5" would indicate maximal gain modulation. It will be appreciated, however, that adjustment of gain modulation is not confined to this specific example and that adjustment of gain modulation can be performed in various ways and have many different settings.

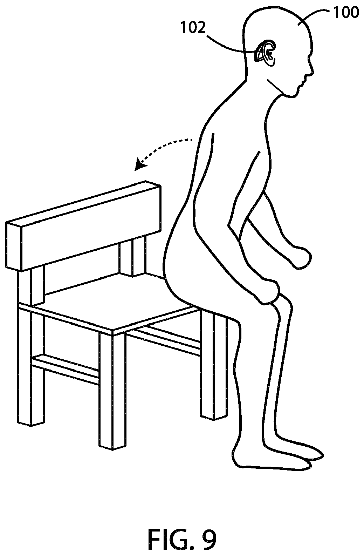

In some cases, other types of data can also be evaluated when identifying chewing. For example, in some cases the ear-worn device ear-worn device system can be configured to evaluate the signals from a motion sensor or other sensor to identify when the device wearer sits down. The process of sitting down includes a characteristic pattern that can be identified from evaluation of a motion sensor signal. Weighting factors for identification of chewing can be adjusted if the system detects that the individual has sat down, since most meals are consumed while individuals are seated. Thus, weighting factors can be changed such that signals from one or more microphones, motion sensors, or other sensors occurring while the device wearer is sitting down are more likely to be deemed to be chewing than are signals from the sensors while the device wearer is standing, walking, or lying down. Referring now to FIG. 9, a schematic view of a device wearer 100 assuming a seated position is shown in accordance with various embodiments herein. Sensors of the hearing assistance device 102 can detect motion associated with sitting down and weighting factors for detecting chewing can be adjusted accordingly. Thus, in various embodiments, the hearing assistance device 102 can be configured to adjust a weighting factor for detection of chewing based on a detected posture. In various embodiments, the hearing assistance device 102 can be configured to adjust a weighting factor for detection of chewing based on detection that the device wearer 100 is seated.

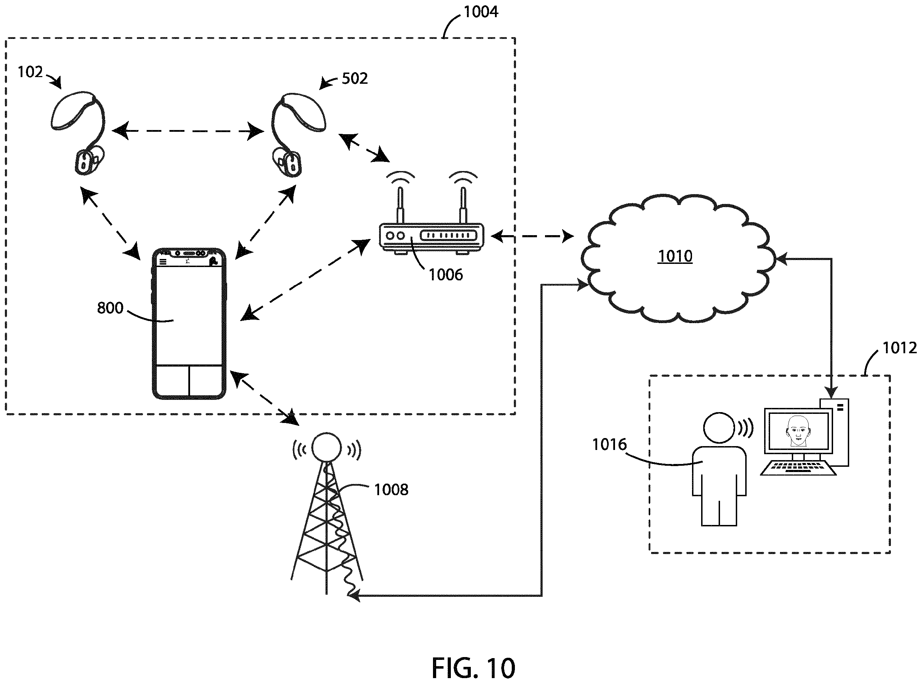

Systems herein can include various components and can be in electronic communication with one another for purposes of exchanging data, sensor signals, notification and/or communications, and the like. Referring now to FIG. 10, a schematic view of components of a hearing assistance system data network is shown in accordance with various embodiments herein. FIG. 10 shows a first hearing assistance device 102 and a second hearing assistance device 502 at a first location or device wearer location 1004. In some embodiments, the system can include and/or can interface with other devices at the first location 1004. The other devices in this example can include an accessory device 800, which could be a smart phone or similar mobile communication/computing device in some embodiments. The accessory device 800 can include sensors and the data and/or signals from the same can be provided to the ear-wearable devices herein. In some embodiments operations described herein can be performed on the ear-wearable devices themselves. In other embodiments, operations described herein can be performed on the accessory device 800 and/or using a cloud computing resource.

FIG. 10 also shows communication equipment including a cell tower 1008 and a network router 1006 to facilitate data communication. FIG. 10 also schematically depicts the cloud 1010 or similar data communication network and/or remote computing resources. The communication equipment can provide data communication capabilities between the hearing assistance devices 102, 502 and other components of the system and/or components such as the cloud 1010 and cloud resources such as a cloud computing resource. In some embodiments, the cloud 1010 and/or resources thereof can host an electronic medical records system. In some embodiments, the cloud 1010 can provide a link to an electronic medical records system.

FIG. 10 also shows a remote location 1012. The remote location 1012 can be the site of a third party 1016, which can be a clinician, care provider, loved one, or the like. The third party 1016 can receive notifications and/or alerts regarding the status of the device wearer. In some embodiments, the third party 1016 can provide an input to turn gain modulation on or off, and/or to increase or decrease the degree of gain modulation.

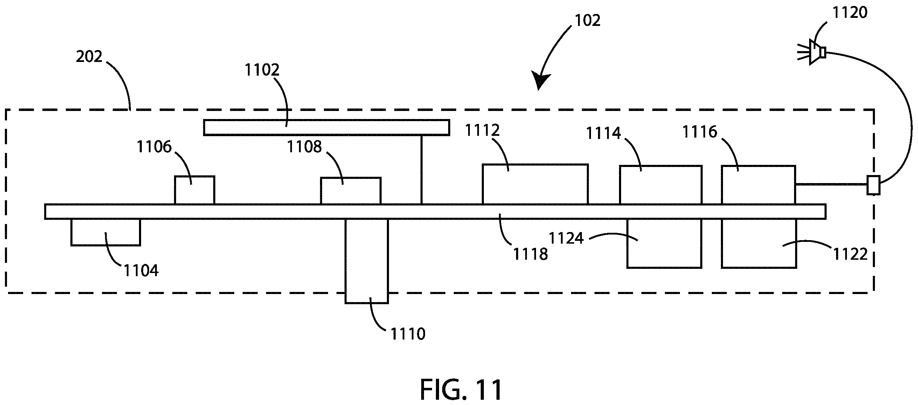

Referring now to FIG. 11, a schematic block diagram is shown with various components of an hearing assistance device 102 in accordance with various embodiments. The block diagram of FIG. 11 represents a generic ear-wearable device for purposes of illustration. The hearing assistance device 102 shown in FIG. 11 includes several components electrically connected to a flexible mother circuit 1118 (e.g., flexible mother board) which is disposed within housing 202. A power supply circuit 1104 can include a battery and can be electrically connected to the flexible mother circuit 1118 and provides power to the various components of the hearing assistance device 102. One or more microphones 1106 are electrically connected to the flexible mother circuit 1118, which provides electrical communication between the microphones 1106 and a digital signal processor (DSP) 1112. Microphones herein can be of various types including, but not limited to, unidirectional, omnidirectional, MEMS based microphones, piezoelectric microphones, magnetic microphones, electret condenser microphones, and the like. Among other components, the DSP 1112 incorporates or is coupled to audio signal processing circuitry such as a sound processor configured to implement various functions described herein. A sensor package 1114 can be coupled to the DSP 1112 via the flexible mother circuit 1118. The sensor package 1114 can include one or more different specific types of sensors such as those described in greater detail below. One or more user switches 1110 (e.g., on/off, volume, mic directional settings) are electrically coupled to the DSP 1112 via the flexible mother circuit 1118. It will be appreciated that the user switches 1110 can extend outside of the housing 202.

An audio output device 1116 is electrically connected to the DSP 1112 via the flexible mother circuit 1118. In some embodiments, the audio output device 1116 comprises a speaker (coupled to an amplifier). In other embodiments, the audio output device 1116 comprises an amplifier coupled to an external receiver 1120 adapted for positioning within an ear of a wearer. The external receiver 1120 can include an electroacoustic transducer, speaker, or loudspeaker. The hearing assistance device 102 may incorporate a communication device 1108 coupled to the flexible mother circuit 1118 and to an antenna 1102 directly or indirectly via the flexible mother circuit 1118. The communication device 1108 can be a BLUETOOTH^{®} transceiver, such as a BLE (BLUETOOTH^{®} low energy) transceiver or other transceiver(s) (e.g., an IEEE 802.11 compliant device). The communication device 1108 can be configured to communicate with one or more external devices, such as those discussed previously, in accordance with various embodiments. In various embodiments, the communication device 1108 can be configured to communicate with an external visual display device such as a smart phone, a video display screen, a tablet, a computer, or the like.

In various embodiments, the hearing assistance device 102 can also include a control circuit 1122 and a memory storage device 1124. The control circuit 1122 can be in electrical communication with other components of the device. In some embodiments, a clock circuit can be in electrical communication with the control circuit. The control circuit 1122 can execute various operations, such as those described herein. In various embodiments, the control circuit 1122 can execute operations resulting in the provision of a user input interface by which the hearing assistance device 102 can receive inputs (including audible inputs, touch based inputs, and the like) from the device wearer. The control circuit 1122 can include various components including, but not limited to, a microprocessor, a microcontroller, an FPGA (field-programmable gate array) processing device, an ASIC (application specific integrated circuit), or the like. The memory storage device 1124 can include both volatile and non-volatile memory. The memory storage device 1124 can include ROM, RAM, flash memory, EEPROM, SSD devices, NAND chips, and the like. The memory storage device 1124 can be used to store data from sensors as described herein and/or processed data generated using data from sensors as described herein.

It will be appreciated that various of the components described in FIG. 11 can be associated with separate devices and/or accessory devices to the ear-wearable device. By way of example, microphones can be associated with separate devices and/or accessory devices. Similarly, audio output devices can be associated with separate devices and/or accessory devices to the ear-wearable device. Further accessory devices as discussed herein can include various of the components as described with respect to an ear-wearable device. For example, an accessory device can include a control circuit, a microphone, a motion sensor, and a power supply, amongst other things.

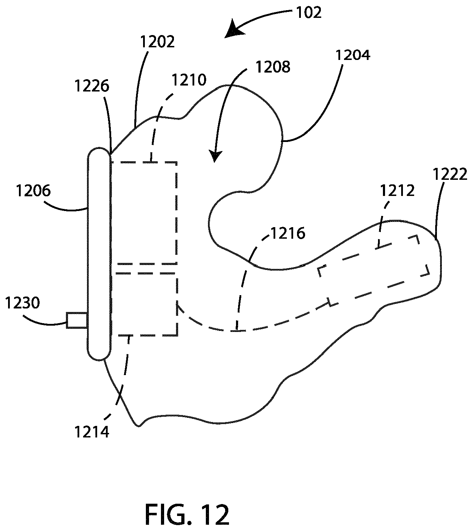

Ear-wearable devices herein can take on many different forms. In some embodiments, the ear-wearable device can be in the form of an in-the-ear style custom ear-wearable device. Referring now to FIG. 12, a schematic view of an in-the-ear style custom hearing assistance device 102 is shown in accordance with various embodiments herein. The hearing assistance device 102 can include an ear-wearable device housing 1202 formed by a shell 1204 and a faceplate 1206. The shell 1204 is custom shaped to mate with the user's ear anatomy (and form a seal with the inner surface of a portion of the ear canal) and defines an internal shell cavity 1208 and a shell aperture at the entrance to the shell cavity 1208. The faceplate 1206 is attached to the shell at the shell aperture to enclose the shell cavity 1208.

The ear-wearable device housing 1202 can define a battery compartment 1210 in which a battery can be disposed to provide power to the device. The hearing assistance device 102 can also include a receiver 1212. The receiver 1212 can include a component that converts electrical impulses into sound, such as an electroacoustic transducer, speaker, or loudspeaker. The housing 1202 can also define a component compartment 1214 that can contain electrical and other components including but not limited to a microphone, a processor, memory, various sensors, one or more communication devices, power management circuitry, and a control circuit. A cable 1216 or connecting wire can include one or more electrical conductors and provide electrical communication between components inside of the component compartment 1214 and components inside of the receiver 1212.

The shell 1204 extends from an ear canal end 1222 to an aperture end 1226. At the aperture end 1226, the shell 1204 defines an aperture that is closed by the faceplate 1206. The faceplate 1206 is sealed to the shell 1204. The faceplate 1206 is shown in FIG. 12 only in a side view but can include many features and structures. A user input device 1230 is shown as part of the faceplate in FIG. 12, and can be a button, lever, switch, dial, or other input device. The faceplate 1206 may also include a battery door, a microphone opening, a pull handle, and other features.

The hearing assistance device 102 shown in FIG. 12 is an in-the-ear style device and thus the shell is designed to be placed within the ear cavity. However, it will be appreciated that many different form factors for ear-wearable devices are contemplated herein. Aspects of ear-wearable devices and functions thereof are described in U.S. Pat. No. 9,848,273; U.S. Publ. Pat. Appl. No. 20180317837; and U.S. Publ. Pat. Appl. No. 20180343527, the content of all of which is herein incorporated by reference in their entirety.

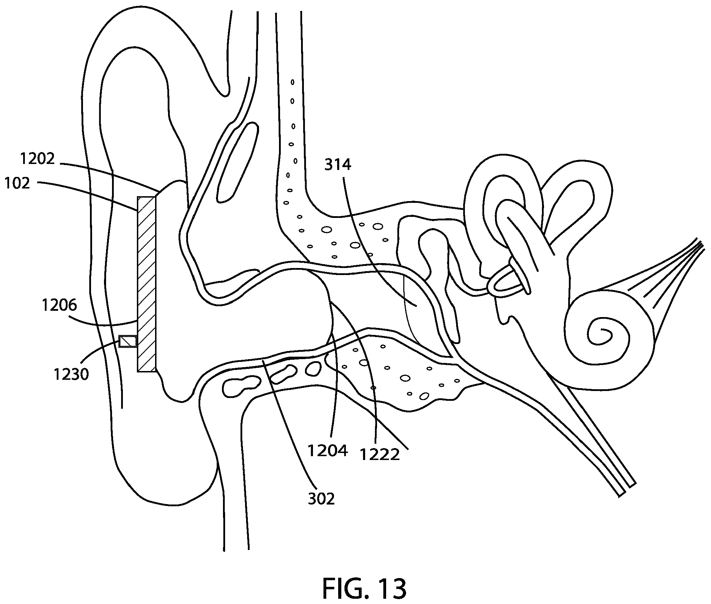

FIG. 13 is a schematic view of an hearing assistance device 102 disposed within the ear of a wearer in accordance with various embodiments herein. The housing 1202 of the hearing assistance device 102 is defined by the shell 1204, which is positioned within the ear canal 302, and the faceplate 1206, which is positioned in the concha. The user input device 1230 on the faceplate 1206 is accessible to be manipulated by the user without having to remove the ear-wearable device from their ear. The ear canal end 1222 of the shell 1204 is positioned close to the user's tympanic membrane 314. Ideally, the shell 1204 fits properly within the user's ear cavity. A proper fit is usually one in which the ear-wearable device forms an acoustic seal with the user's ear cavity, so that it is contacting the ear cavity around a circumference of the ear-wearable device at some location on the shell 1204 of the hearing assistance device 102. A proper fit is also comfortable to the user, so that the shell 1204 does not put too much pressure on the walls of the ear canal 302 or features of the concha. The receiver 1212 (FIG. 12) is positioned within the shell 1204 at the ear canal end 1222 of the shell 1204 to minimize the distance between the receiver 1212 and the tympanic membrane 314 without physically contacting the tympanic membrane 314.

### Methods

Many different methods are contemplated herein, including, but not limited to, methods of making, methods of using, and the like. Aspects of system/device operation described elsewhere herein can be performed as operations of one or more methods in accordance with various embodiments herein.

In various embodiments, operations described herein and method steps can be performed as part of a computer-implemented method executed by one or more processors of one or more computing devices. In various embodiments, operations described herein and method steps can be implemented instructions stored on a non-transitory, computer-readable medium that, when executed by one or more processors, cause a system to execute the operations and/or steps.

In an embodiment, a method of dynamically adjusting gain values for a hearing assistance device is included. The method can include outputting sound to a wearer of a wearer of a hearing assistance device based on sound detected with a microphone. The method can further include detecting chewing of the device wearer by evaluating signals from the microphone and/or the motion sensor. The method can further include modulating a gain value of a sound processor for sounds output through the receiver based on the chewing detected.

In an embodiment, the method can further include reducing the gain value of the sound processor for sounds detected with the microphone and then output through the receiver based on the chewing detected.

In an embodiment, the method can further include receiving an input regarding a degree of gain value reduction when the chewing is detected.

In an embodiment, the method can further include adjusting the gain value of the sound processor greater than 1, 5, 10, 15, 20, or 30 times per second.

In an embodiment, the method can further include detecting chewing of the wearer by detecting a predetermined pattern within signals from the microphone and/or the motion sensor. In an embodiment, the method can further include executing a binary classification for the chewing detection on a per time unit basis.

In an embodiment, the method can further include adjusting a weighting factor for detection of the chewing based on a detected posture. In an embodiment, the method can further include adjusting a weighting factor for detection of the chewing based on detecting that the device wearer is seated.

In an embodiment, the method can further include characterizing an ambient sound environment and setting a degree of gain value modulation based on the same. For example, in a sound environment with relatively loud ambient sound, the degree of gain value modulation can be set to be higher as it is likely that the microphone may be picking up more sound that can lead to more sound volume being output through the speaker that can result in more feedback issues.

### Sensors

Hearing assistance devices and/or systems herein can include one or more sensor packages (including one or more discrete or integrated sensors) to provide data. The sensor package can comprise one or a multiplicity of sensors. In some embodiments, the sensor packages can include one or more motion sensors (or movement sensors) amongst other types of sensors. Motion sensors herein can include inertial measurement units (IMU), accelerometers, gyroscopes, barometers, altimeters, and the like. The IMU can be of a type disclosed in commonly owned U.S. Patent No. 9,848,273, filed October 21, 2016, which is incorporated herein by reference. As used herein the term "inertial measurement unit" or "IMU" shall refer to an electronic device that can generate signals related to a body's specific force and/or angular rate. IMUs herein can include one or more accelerometers (3, 6, or 9 axis) to detect linear acceleration and a gyroscope to detect rotational rate. In some embodiments, an IMU can also include a magnetometer to detect a magnetic field.

In some embodiments, the motion sensors can be disposed in a fixed position with respect to the head of a device wearer, such as worn on or near the head or ears. In some embodiments, the operatively connected motion sensors can be worn on or near another part of the body such as on a wrist, arm, or leg of the device wearer.

According to various embodiments, the sensor package can include one or more of an IMU, and accelerometer (3, 6, or 9 axis), a gyroscope, a barometer, an altimeter, a magnetometer, a magnetic sensor, an eye movement sensor, a pressure sensor, an acoustic sensor, a telecoil, a heart rate sensor, a global positioning system (GPS), a temperature sensor, a blood pressure sensor, an oxygen saturation sensor, an optical sensor, a blood glucose sensor (optical or otherwise), a galvanic skin response sensor, a cortisol level sensor (optical or otherwise), a microphone, acoustic sensor, an electrocardiogram (ECG) sensor, electroencephalography (EEG) sensor which can be a neurological sensor, eye movement sensor (e.g., electrooculogram (EOG) sensor), myographic potential electrode sensor (EMG), a heart rate monitor, a pulse oximeter or oxygen saturation sensor (SpO2), a wireless radio antenna, blood perfusion sensor, hydrometer, sweat sensor, cerumen sensor, air quality sensor, pupillometry sensor, cortisol level sensor, hematocrit sensor, light sensor, image sensor, and the like.

In some embodiments, spatial location sensors and/or geolocation sensors can be included and can take the form of an integrated circuit that can include components for receiving signals from GPS, GLONASS, BeiDou, Galileo, SBAS, WLAN, BT, FM, and/or NFC type protocols

In some embodiments, the sensor package can be part of a hearing assistance device. However, in some embodiments, the sensor packages can include one or more additional sensors that are external to a hearing assistance device. For example, various of the sensors described above can be part of a wrist-worn or ankle-worn sensor package, or a sensor package supported by a chest strap. In some embodiments, sensors herein can be disposable sensors that are adhered to the device wearer ("adhesive sensors") and that provide data to the hearing assistance device or another component of the system.

Data produced by the sensor(s) of the sensor package can be operated on by a processor of the device or system.

It will be appreciated that the sensor package can include one or more sensors that are external to the hearing assistance device. In addition to the external sensors discussed hereinabove, the sensor package can comprise a network of body sensors (such as those listed above) that sense movement of a multiplicity of body parts (e.g., arms, legs, torso).

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

All publications and patent applications in this specification are indicative of the level of ordinary skill in the art to which this invention pertains. All publications and patent applications are herein incorporated by reference to the same extent as if each individual publication or patent application was specifically and individually indicated by reference.

As used herein, the recitation of numerical ranges by endpoints shall include all numbers subsumed within that range (e.g., 2 to 8 includes 2.1, 2.8, 5.3, 7, etc.).

The headings used herein are provided for consistency with suggestions under 37 CFR 1.77 or otherwise to provide organizational cues. These headings shall not be viewed to limit or characterize the invention(s) set out in any claims that may issue from this disclosure. As an example, although the headings refer to a "Field," such claims should not be limited by the language chosen under this heading to describe the so-called technical field. Further, a description of a technology in the "Background" is not an admission that technology is prior art to any invention(s) in this disclosure. Neither is the "Summary" to be considered as a characterization of the invention(s) set forth in issued claims.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the spirit and scope herein.

The invention may be defined by the following aspects:
1. A hearing assistance device comprising:
   a control circuit;
   a microphone, wherein the microphone is in electrical communication with the control circuit;
   a motion sensor, wherein the motion sensor is in electrical communication with the control circuit;
   a receiver, wherein the receiver is in electrical communication with the control circuit; and
   a sound processor, wherein the sound processor is in electrical communication with the control circuit;
   wherein the hearing assistance device is configured to
      output sound to a wearer of the hearing assistance device with the receiver based on sound detected with the microphone;
      detect chewing of the wearer by evaluating signals from the microphone and/or the motion sensor; and
      modulate a gain value of the sound processor for sounds output through the receiver based on the chewing detected.
2. The hearing assistance device of aspect 1, wherein the hearing assistance device is configured to reduce the gain value of the sound processor for sounds detected with the microphone and then output through the receiver based on the chewing detected.
3. The hearing assistance device of aspect 1 or 2, wherein the hearing assistance device is configured to receive an input regarding a degree of gain value reduction when the chewing is detected.
4. The hearing assistance device of any of the preceding aspects, wherein the hearing assistance device is configured to adjust the gain value of the sound processor greater than 20 times per second.
5. The hearing assistance device of any of any of the preceding aspects, wherein the hearing assistance device is configured to detect chewing of the wearer by detecting a predetermined pattern within signals from the microphone and/or the motion sensor.
6. The hearing assistance device of any of the preceding aspects, wherein the hearing assistance device is configured to execute a binary classification for the chewing detection on a per time unit basis.
7. The hearing assistance device of any of the preceding aspects, wherein the hearing assistance device is configured to adjust a weighting factor for detection of the chewing based on a detected posture.
8. The hearing assistance device of any of the preceding aspects, wherein the hearing assistance device is configured to adjust a weighting factor for detection of the chewing based on detection that the device wearer is seated.
9. The hearing assistance device of any of the preceding aspects, wherein the hearing assistance device is configured to characterize an ambient sound environment and set a degree of gain value modulation based on the same.
10. The hearing assistance device of any of the preceding aspects, the sound processor comprising an amplifier.
11. The hearing assistance device of any of the preceding aspects, the sound processor comprising a digital signal processor.
12. The hearing assistance device of any of the preceding aspects, the motion sensor comprising an accelerometer.
13. The hearing assistance device of any of the preceding aspects, the receiver comprising a speaker.
14. The hearing assistance device of any of the preceding aspects, the hearing assistance device comprising at least one selected from the group consisting of a RIC hearing aid and a custom hearing aid.
15. A hearing-assistance system comprising:
   a first hearing assistance device, the first hearing assistance device comprising
      a first control circuit;
      a first microphone, wherein the first microphone is in electrical communication with the first control circuit;
      a first motion sensor, wherein the first motion sensor is electrical communication with the first control circuit; and
      a first speaker, wherein the first speaker is in electrical communication with the first control circuit;
   a second hearing assistance device, the second hearing assistance device comprising
      a second control circuit, wherein the second control circuit is in electrical communication with the second control circuit;
      a second microphone, wherein the second microphone is in electrical communication with the second control circuit;
      a second motion sensor, wherein the second motion sensor is in electrical communication with the second control circuit; and
      a second speaker, wherein the second speaker is in electrical communication with the second control circuit;
   wherein the hearing-assistance system is configured to
      output sound to a wearer of the hearing-assistance system based on sound detected with the first microphone and/or the second microphone;
      detect chewing of a device wearer by evaluating signals from the microphones and/or the motion sensors; and
      modulate a gain value for sounds detected with the microphones and then output through the speakers based on the detected chewing.
16. The hearing-assistance system of aspect 15, wherein the hearing-assistance system is configured to reduce the gain value for sounds detected with the first microphone and/or the second microphone and then output through the first speaker and/or the second speaker based on the chewing detected.
17. The hearing-assistance system of any of aspects 15 or 16, wherein the hearing-assistance system is configured to receive an input regarding a degree of gain value reduction when the chewing is detected.
18. The hearing-assistance system of any of aspects 15-17, wherein the hearing-assistance system is configured to adjust the gain value greater than 20 times per second.
19. The hearing-assistance system of any of aspects 15-18, wherein the hearing-assistance system is configured to detect chewing of the wearer by detecting a predetermined pattern within signals from the microphones and/or the motion sensors.
20. The hearing-assistance system of any of aspects 15-19, wherein the hearing-assistance system is configured to execute a binary classification for the chewing detection on a per time unit basis.
21. The hearing-assistance system of any of aspects 15-20, wherein the hearing-assistance system is configured to adjust a weighting factor for detection of the chewing based on a detected posture.
22. The hearing-assistance system of any of aspects 15-22, wherein the hearing-assistance system is configured to adjust a weighting factor for detection of the chewing based on detection that the device wearer is seated.
23. The hearing-assistance system of any of aspects 15-22, wherein the hearing-assistance system is configured to characterize an ambient sound environment and set a degree of gain value modulation based on the same.
24. The hearing-assistance system of any of aspects 15-23, wherein gain values for the first hearing assistance device and the second hearing assistance device are adjusted independently.
25. The hearing-assistance system of any of aspects 15-24, the hearing-assistance system comprising at least one selected from the group consisting of a RIC hearing aid and a custom hearing aid.
26. A method of dynamically adjusting gain values for a hearing assistance device comprising:
   outputting sound to a wearer of a wearer of a hearing assistance device based on sound detected with a microphone;
   detecting chewing of the device wearer by evaluating signals from the microphone and/or the motion sensor; and
   modulating a gain value of a sound processor for sounds output through the receiver based on the chewing detected.
27. The method of aspect 26, further comprising reducing the gain value of the sound processor for sounds detected with the microphone and then output through the receiver based on the chewing detected.
28. The method of aspect 26 or 27, further comprising receiving an input regarding a degree of gain value reduction when the chewing is detected.
29. The method of any of aspects 26-28, further comprising adjusting the gain value of the sound processor greater than 20 times per second.
30. The method of any of aspects 26-29, further comprising detecting chewing of the wearer by detecting a predetermined pattern within signals from the microphone and/or the motion sensor.
31. The method of any of aspects 26-30, further comprising executing a binary classification for the chewing detection on a per time unit basis.
32. The method of any of aspects 26-31, further comprising adjusting a weighting factor for detection of the chewing based on a detected posture.
33. The method of any of aspects 26-32, further comprising adjusting a weighting factor for detection of the chewing based on detecting that the device wearer is seated.
34. The method of any of aspects 26-33, further comprising characterizing an ambient sound environment and setting a degree of gain value modulation based on the same.

## Claims

1. A hearing assistance device comprising:
a control circuit;
a microphone, wherein the microphone is in electrical communication with the control circuit;
a motion sensor, wherein the motion sensor is in electrical communication with the control circuit;
a receiver, wherein the receiver is in electrical communication with the control circuit; and
a sound processor, wherein the sound processor is in electrical communication with the control circuit;
wherein the hearing assistance device is configured to
output sound to a wearer of the hearing assistance device with the receiver based on sound detected with the microphone;
detect chewing of the wearer by evaluating signals from the microphone and/or the motion sensor; and
modulate a gain value of the sound processor for sounds output through the receiver based on the chewing detected.

2. The hearing assistance device of claim 1,
wherein the hearing assistance device is configured to reduce the gain value of the sound processor for sounds detected with the microphone and then output through the receiver based on the chewing detected; and/or
wherein the hearing assistance device is configured to receive an input regarding a degree of gain value reduction when the chewing is detected.

3. The hearing assistance device of any of the preceding claims, wherein the hearing assistance device is configured to adjust the gain value of the sound processor greater than 20 times per second.

4. The hearing assistance device of any of any of the preceding claims, wherein the hearing assistance device is configured to detect chewing of the wearer by detecting a predetermined pattern within signals from the microphone and/or the motion sensor.

5. The hearing assistance device of any of the preceding claims, wherein the hearing assistance device is configured to execute a binary classification for the chewing detection on a per time unit basis.

6. The hearing assistance device of any of the preceding claims, wherein the hearing assistance device is configured to adjust a weighting factor for detection of the chewing based on a detected posture.

7. The hearing assistance device of any of the preceding claims, wherein the hearing assistance device is configured to adjust a weighting factor for detection of the chewing based on detection that the device wearer is seated.

8. The hearing assistance device of any of the preceding claims, wherein the hearing assistance device is configured to characterize an ambient sound environment and set a degree of gain value modulation based on the same.

9. The hearing assistance device of any of the preceding claims,
the sound processor comprising an amplifier; and/or
the hearing assistance device of any of the preceding claims, the sound processor comprising a digital signal processor.

10. The hearing assistance device of any of the preceding claims, the motion sensor comprising an accelerometer.

11. A hearing-assistance system comprising:
a first hearing assistance device, the first hearing assistance device comprising
a first control circuit;
a first microphone, wherein the first microphone is in electrical communication with the first control circuit;
a first motion sensor, wherein the first motion sensor is electrical communication with the first control circuit; and
a first speaker, wherein the first speaker is in electrical communication with the first control circuit;
a second hearing assistance device, the second hearing assistance device comprising
a second control circuit, wherein the second control circuit is in electrical communication with the second control circuit;
a second microphone, wherein the second microphone is in electrical communication with the second control circuit;
a second motion sensor, wherein the second motion sensor is in electrical communication with the second control circuit; and
a second speaker, wherein the second speaker is in electrical communication with the second control circuit;
wherein the hearing-assistance system is configured to
output sound to a wearer of the hearing-assistance system based on sound detected with the first microphone and/or the second microphone;
detect chewing of a device wearer by evaluating signals from the microphones and/or the motion sensors; and
modulate a gain value for sounds detected with the microphones and then output through the speakers based on the detected chewing;
preferably wherein gain values for the first hearing assistance device and the second hearing assistance device are adjusted independently.

12. A method of dynamically adjusting gain values for a hearing assistance device comprising:
outputting sound to a wearer of a wearer of a hearing assistance device based on sound detected with a microphone;
detecting chewing of the device wearer by evaluating signals from the microphone and/or the motion sensor; and
modulating a gain value of a sound processor for sounds output through the receiver based on the chewing detected.

13. The method of claim 12,
further comprising reducing the gain value of the sound processor for sounds detected with the microphone and then output through the receiver based on the chewing detected; and/or
further comprising receiving an input regarding a degree of gain value reduction when the chewing is detected.

14. The method of claim 12 or 13, further comprising adjusting the gain value of the sound processor greater than 20 times per second.

15. The method of any of claims 12-14,
further comprising detecting chewing of the wearer by detecting a predetermined pattern within signals from the microphone and/or the motion sensor; and/or
further comprising executing a binary classification for the chewing detection on a per time unit basis; and/or
further comprising adjusting a weighting factor for detection of the chewing based on a detected posture; and/or
further comprising adjusting a weighting factor for detection of the chewing based on detecting that the device wearer is seated; and/or
further comprising characterizing an ambient sound environment and setting a degree of gain value modulation based on the same.
